# EUROPÄISCHE PATENTSCHRIFT

(11) **EP 4 027 934 B1**
(45) Veröffentlichungstag und Bekanntmachung des Hinweises auf die Patenterteilung: **05.07.2023**
(21) Anmeldenummer: 20786462.0
(22) Anmeldetag: 10.09.2020
(51) Int. Cl.: A61C 7/00, A61F 5/01

(54) **VERFAHREN ZUM BERECHNEN EINES STIMULUS FÜR DIE FORMÄNDERUNG EINES MEDIZINISCHEN HILFSMITTELS**
METHOD FOR CALCULATING A STIMULUS FOR THE CHANGE OF SHAPE OF A MEDICAL AID
PROCÉDÉ DE CALCUL D'UN STIMULUS POUR MODIFIER LA FORME D'UN INSTRUMENT MÉDICAL

(30) Priorität: 11.09.2019 DE 102019124364
(43) Veröffentlichungstag der Anmeldung: 20.07.2022
(73) Patentinhaber: K Line Europe GmbH, 40474 Düsseldorf (DE)
(72) Erfinder: KANDIL, Sherif, 40474 Düsseldorf (DE); EL BANNA, Ahmed Khaled, Nasr City Kairo, 11371 (EG)
(74) Vertreter: Kalkoff & Partner Patentanwälte mbB
(86) Internationale Anmeldenummer: PCT/EP2020/075409
(87) Internationale Veröffentlichungsnummer: WO 2021/048318

(56) Entgegenhaltungen:
- WO-A1-2017/079157
- US-A1- 2001 007 738

## Beschreibung

Die Erfindung betrifft ein Verfahren zum Berechnen eines Stimulus für die Formänderung eines medizinischen Hilfsmittels und eine Steuereinheit.

Medizinische Hilfsmittel umfassen zahlreiche Produkte, die sämtlich eingesetzt werden, um körperliche oder organische Defekte oder Krankheiten zu reduzieren oder zu heilen. Es handelt sich dabei um Hilfsmittel, die im oder am Körper oder in Verbindung mit anderen medizinischen Hilfsmitteln wie Schienen, insbesondere Zahnkorrekturvorrichtungen, Orthesen, Extendern, Fixateuren, Implantaten oder dergleichen eingesetzt werden. Die medizinischen Hilfsmittel bestehen in der Regel aus einem Polymer oder einer Kombination von Polymeren oder aus einem Metall bzw. einer Kombination oder Legierung von Metallen, die unter dem Einfluss eines Stimulus ihre Gestalt in vorberechneter Weise ändern oder sie weisen solches Material auf. Zahlreiche medizinische Behandlungen setzen eine schrittweise Änderung von Knochen- oder Zahnstellung, von Organen oder Gewebe voraus. Diese schrittweise Änderung muss präzise erfolgen.

So sind z. B. Zahnkorrekturvorrichtungen aus der WO 2016/193424 A1 und der WO 2017/079157 A1 bekannt, die unter dem Einfluss eines Stimulus ihre Form ändern und dadurch die Stellung eines Zahns von einer Ist-Stellung bis hin zu einer endgültigen Soll-Stellung ändern. Diese Zahnkorrekturvorrichtungen (im Folgenden auch kurz "Korrekturvorrichtungen") bestehen in der Regel aus einem Polymer oder einer Kombination von Polymeren oder aus einem Metall bzw. einer Kombination oder Legierung von Metallen, die unter dem Einfluss eines Stimulus ihre Gestalt in vorberechneter Weise ändern. Meist erfolgt eine schrittweise Korrektur des Zahns, der eine Folge von einzelnen Formänderungen des medizinischen Hilfsmittels voraussetzt. Für andere medizinische Hilfsmittel, die z. B. bei der Korrektur von Knochenbrüchen, Knochenverlängerungen oder der Wundheilung eingesetzt werden, sind ebenfalls schrittweise ablaufende Behandlungen bekannt.

Nach aktuellem Stand der Technik wird, wiederum beispielsweise bei einer Zahnkorrektur, ausgehend von der ursprünglichen Zahnstellung, ein medizinisches Hilfsmittel, hier eine Zahnkorrekturvorrichtung, entworfen, die unter dem Einfluss eines ersten Stimulus eine erste Formänderung erfährt und die dann in einem ersten Korrekturschritt eine erste Änderung der Zahnstellung bewirkt. Oft schließt sich dann ein zweiter Korrekturschritt an, indem ein weiterer Stimulus auf das medizinische Hilfsmittel einwirkt und eine zweite Formänderung auslöst. Dieser Vorgang kann wiederholt werden. Entsprechend wird z. B. bei der Korrektur von Knochenverletzungen, bei der Knochenverlängerung oder der Wundheilung vorgegangen.

Es hat sich jedoch herausgestellt, dass die Ist-Stellung eines zu korrigierenden Zahns oder Knochens bzw. einer Wunde nach dem ersten Korrekturschritt oft nicht mit der erwarteten Soll-Stellung übereinstimmt, sei es, dass ein Zahn, ein Knochen oder eine Wunde sich als beweglicher oder unbeweglicher erweist als angenommen oder sei es, dass das medizinische Hilfsmittel sich nicht wie vorherberechnet in seiner Form verändern lässt.

Es ist daher Aufgabe der Erfindung, eine optimierte Behandlung für den Patienten zu erreichen.

Diese Aufgabe wird gelöst mit einem Verfahren nach Anspruch 1 und mit einer Steuereinheit nach Anspruch 11.

Das erfindungsgemäße Verfahren zum Berechnen eines Stimulus für die Formänderung eines medizinischen Hilfsmittels, um die Stellung eines medizinischen Hilfsmittels von einer Ist-Stellung bis zu einem endgültigen Soll-Zustand zu ändern, weist folgende Schritte auf:
- Erfassen der Ist-Stellung des zu korrigierenden Körperteils nach einem ersten Korrekturschritt,
- Eingeben der Ist-Stellung des zu korrigierenden Körperteils in eine Steuereinheit,

- Vergleichen der Ist-Stellung des zu korrigierenden Körperteils mit einer Soll-Stellung, die in der Steuereinheit gespeichert ist,
- ggf. Erfassen der Abweichung von Ist- und Soll-Stellung des zu korrigierenden Körperteils durch die Steuereinheit,
- Berechnen des Stimulus, der auf das medizinische Hilfsmittel zum Ausführen eines zweiten Korrekturschritts einzuwirken hat, unter Berücksichtigung der Abweichung von Ist- und Soll-Stellung des zu korrigierenden Körperteils durch die Steuereinheit, wobei in der Steuereinheit die Wirkung des Stimulus auf das medizinische Hilfsmittel hinterlegt ist.

Körperteile, die einer Korrektur bedürfen, können ein Zahn, ein Knochen, ein Organ oder Gewebe sein. Das medizinische Hilfsmittel kann eine Schiene, insbesondere eine Zahnkorrekturvorrichtung, eine Orthese, ein Extender beispielsweise zum Dehnen oder zum Verlängern von Knochen, ein Fixateur z. B. zum Ausrichten von Knochen, ein Implantat, chirurgisches Nahtmaterial oder Verbandsmaterial oder dergleichen oder ein Teil eines solchen medizinischen Hilfsmittels sein. Die Korrektur der Stellung eines Körperteils kann sich über Wochen, Monate, manchmal auch Jahre hinziehen, wie z. B. für Zahnkorrekturen bekannt ist. Ein Behandlungsplan von der Ausgangsstellung des beispielsweise zu korrigierenden Zahns oder Knochens bis zur endgültigen Soll-Stellung ist üblicherweise in einzelne Behandlungsschritte gegliedert, die als Korrekturschritte aufeinander folgen. Jeder Korrekturschritt beginnt mit einer Formänderung des medizinischen Hilfsmittels. Hierzu ist es erforderlich, einen Stimulus zu berechnen, der die gewünschte Formänderung herbeiführt. Die Behandlungsschritte umfassen einen Hinweis, in welchen Zeitabständen Formänderungen für den jeweils nächsten Korrekturschritt vorzunehmen sind. Soweit im Zusammenhang mit dieser Erfindung Bezug auf einen Zahn oder Knochen genommen wird, gelten sämtliche Ausführungen auch für das Korrigieren der Zahnstellung mehrerer Zähne bzw. von Kiefer-Fehlstellungen oder Knochen.

Das Erfassen der Ist-Stellung nach einem ersten Korrekturschritt kann konventionell analog, z. B. durch einen Abdruck, erfolgen, bevorzugt wird die Ist-Stellung jedoch durch digitale Vermessung erfasst, z. B. mittels digitaler Photographie. Alternativ kann die Ist-Stellung jedoch auch durch einen Sensor erfasst werden, der die aktuelle Ist-Stellung des zu korrigierenden Zahns bzw. der zu korrigierenden Zähne meldet. Der Sensor kann in einer vorteilhaften Ausführung der Erfindung an das medizinische Hilfsmittel angebracht sein, der Sensor kann aber auch an einem Scanner angebracht sein. Bei Zahnkorrekturen wird bevorzugt ein intraoraler Scanner eingesetzt. Die analog oder digital erfasste Ist-Stellung wird dann in eine Steuereinheit eingegeben. Die Eingabe kann durch manuelle Eingabe der Daten in eine Steuereinheit erfolgen. Bei digital vorliegenden Daten kann die Eingabe unmittelbar und automatisiert erfolgen, beispielsweise dadurch, dass an der Steuereinheit ein Datenabgleich initialisiert und durchgeführt wird. Vorteilhaft erfolgt die Eingabe an einem Eingabe-Portal, insbesondere einem Eingabe-Portal des Herstellers des medizinischen Hilfsmittels oder einem Eingabe-Portal des behandelnden Arztes, z. B. einem Bildschirm oder einem Lesegerät, das Daten für eine dreidimensionale Positionsbeschreibung eines Zahns erfassen kann. Das Eingabe-Portal übermittelt die Eingaben an die Steuereinheit. Das Eingabe-Portal kann auf einem Computer angezeigt werden, es kann mittels Anwendungssoftware (App) auf einem Smartphone angezeigt werden, es kann aber auch als eigenes Gerät speziell ausgebildet sein, allein zu dem Zweck, Eingaben zur Ist-Stellung vorzunehmen.

Optional können Daten des Patienten in das Eingabe-Portal eingegeben werden, um auch die Befindlichkeit des Patienten in die Optimierung der korrigierenden medizinischen Behandlung mittels des medizinischen Hilfsmittels einfließen zu lassen. Diese Daten können das Trageverhalten des Patienten bezüglich des medizinischen Hilfsmittels betreffen, Verhaltensmuster der Verwendung des medizinischen Hilfsmittels durch den Patienten, seine Bereitwilligkeit, den Behandlungsvorschriften zu folgen, den Behandlungsfortschritt, aber auch eine Vielzahl von Eingaben betreffend Vitalzeichen, Blutwerte, Angaben zur Speichelzusammensetzung und -qualität bzw. -quantität, Hormonstatus oder andere Analysedaten, z. B. zu Ausscheidungen des Patienten.

Bevorzugt werden die Daten in der Weise eingegeben, dass sie von einem Algorithmus bzw. einer Software einfach verarbeitet werden können, z. B. in Bewertungsstufen wie "niedrig" - "mittel" - "hoch" oder in einer Bewertung nach Noten, z. B. von 1 bis 5. Es ist bevorzugt, dass das Eingabe-Portal zu diesen Eingaben die Bewertungsstufen anzeigt, so dass der Patient oder der behandelnde Arzt schnell und einfach auswählen und eingeben kann. Es können auch Daten erfasst werden, die z. B. von Sensoren an dem medizinischen Hilfsmittel erfasst werden, z. B. betreffend die Zeiträume, in denen der Patient das medizinische Hilfsmittel trägt bzw. nutzt oder zur Art und Weise, wie der Patient das medizinische Hilfsmittel trägt bzw. nutzt.

Die Steuereinheit ist zur Verarbeitung von Software bzw. von Algorithmen ausgelegt. Vereinfachend werden nachfolgend Berechnungen, die mittels Software erfolgen, bzw. das Abarbeiten von Algorithmen in der Steuereinheit als Tätigkeit bzw. Betätigung der Steuereinheit beschrieben.

In der Steuereinheit ist für das medizinische Hilfsmittel eine Soll-Stellung für jeden einzelnen Korrekturschritt der Behandlung bis hin zur endgültigen Soll-Stellung hinterlegt. Anschließend an die Eingabe der Ist-Stellung nach dem ersten Korrekturschritt erfolgt ein Vergleichen der Ist-Stellung mit der Soll-Stellung des zu korrigierenden Körperteils durch die Steuereinheit. Eine etwa ermittelte Abweichung der Ist-Stellung von der Soll-Stellung wird nach Eingabe durch die Steuereinheit erfasst.

Auf der Grundlage des Vergleichs der Ist-Stellung mit der Soll-Stellung wird der Stimulus berechnet, der erforderlich ist, um das medizinische Hilfsmittel zu der für einen zweiten Korrekturschritt erforderlichen Formänderung zu veranlassen. Optional wird berechnet, welche Abschnitte des medizinischen Hilfsmittels einem Stimulus auszusetzen sind, um eine Formänderung zu erreichen. Dabei wird eine etwa ermittelte Abweichung der Ist-Stellung von der Soll-Stellung berücksichtigt: Wurde z. B. der zu korrigierende Zahn oder Knochen weiter bewegt als ursprünglich vorgesehen oder wenn der Patient die schnelle Bewegung des Zahns oder Knochens nicht verträgt, kann der Stimulus für den zweiten Korrekturschritt schwächer ausfallen oder es kann ein anderer, schwächer wirkender Stimulus gewählt werden, weil nur noch eine geringere Korrekturbewegung erforderlich ist. Wurde der zu korrigierende Zahn oder Knochen weniger weit bewegt als ursprünglich vorgesehen oder signalisiert der Patient, dass er eine schnellere Bewegung des Zahns oder Knochens wünscht, kann ein stärkerer Stimulus oder ein anderer, stärker wirkender Stimulus eingesetzt werden. Gleiches gilt für die Behandlung von Narben oder die Behandlung von Brandwunden oder von Hautkorrekturen. Optional werden also auch die Befindlichkeit des Patienten bzw. die Eingaben zum Befinden des Patienten bei der Berechnung des Stimulus für den nächsten Korrekturschritt berücksichtigt. Auch die Zeiträume, in denen der Patient das medizinische Hilfsmittel trägt, können in die Berechnung des Stimulus einfließen. Alternativ kann eine Formveränderung des medizinischen Hilfsmittels auch jeweils durch eine Kombination von zwei oder mehr Stimuli bewirkt werden. Auf diese Weise wird erreicht, dass die Korrektur des Körperteils (Zahn, Knochen, Haut, Gewebe, Organ) effizient, also schnell und mit geringer Belastung des Patienten erfolgt.

Die hier beispielsweise beschriebene Zahnkorrekturvorrichtung kann eine Schiene sein, die auf einen oder mehrere Zähne aufgesetzt wird. Sie kann auch als Draht ausgebildet sein, der z. B. mittels Halterungen auf den oder die zu korrigierenden Zähne aufgesetzt wird. Das medizinische Hilfsmittel allgemein kann aus beliebigem Material hergestellt sein. Bevorzugt weist es ausschließlich dynamisches synthetisches Material auf. Es kann aber vorteilhaft auch ein medizinisches Hilfsmittel sein, das aus einer Kombination von dynamischem synthetischem Material mit herkömmlichem, statischem synthetischem Material besteht, wobei das statische synthetische Material in seiner Form nicht durch einen Stimulus geändert werden kann (ausgenommen mechanische Kraft). Weiter kann synthetisches Material, also dynamisches Material oder dynamisches Material in Kombination mit statischem Kunststoffmaterial, mit einem anderen Werkstoff, typisch z. B. einer Kunststoff-Metall-Kombination, eingesetzt werden. Nach einer weiteren Alternative kann das synthetische Material mit natürlichem oder synthetischen Gummi oder Kautschuk kombiniert werden. Bevorzugt tragen alle verwendeten Werkstoffe dazu bei, Zahn oder Knochen bzw. Gewebe oder Organ zu richten, d. h., in die endgültige Soll-Stellung zu überführen. Das erfindungsgemäße Verfahren wird mit einem medizinischen Hilfsmittel durchgeführt, das unter Verwendung eines dynamischen, synthetischen Materials hergestellt ist, das unter Einwirkung eines Stimulus seine Form in berechenbarer Weise ändert. Die Formänderung des medizinischen Hilfsmittels bewirkt eine Bewegung des zu korrigierenden Körperteils von einer Ist-Stellung in eine Soll-Stellung.

Ein solches Material, in der Regel ein thermoplastischer Kunststoff, besteht zum Beispiel aus zwei unterschiedlichen Polymeren, die aneinander anliegen, vorzugsweise schichtweise aneinander anliegen. Dabei reagiert ein erstes Polymer nicht auf bewegungsinduzierende Stimuli, während das zweite Polymer auf bewegungsinduzierende Reize reagiert, insbesondere durch Form- und/oder Volumenveränderung. Bewegungsinduzierende Stimuli können der Kontakt mit Feuchtigkeit bzw. Flüssigkeit, insbesondere Wasser und wasserhaltige Flüssigkeiten, Temperatur, insbesondere Wärme und/oder Strahlung sein. Solche Kunststoffe werden auch als Polymere mit Gestalterinnerungsvermögen (shape memory polymers SMP) bezeichnet. Alternativ können für das medizinische Hilfsmittel auch Metalle oder Metalllegierungen mit Gestalterinnerungsvermögen (shape memory alloys SMA) eingesetzt werden.

Als Stimulus wirken viele Reize, die jeweils abgestimmt werden auf die Materialeigenschaften der Zahnkorrekturvorrichtung, um eine gewünschte Verformung zu bewirken. Ein Stimulus wird insbesondere ausgewählt aus der Gruppe, die die Parameter Zeit, Temperatur, pH-Wert, Strom bzw. elektrische Spannung, Strahlung, insbesondere Infrarotstrahlung, Schall, Belichtung und Flüssigkeit, z. B. Wasser oder Lösungsmittel, umfasst. Alternativ ist der Stimulus ausgewählt aus einer Kombination dieser Parameter, z. B. Temperatur und pH-Wert, Wasser und Temperatur oder Zeit und Temperatur. Ein Stimulus kann aber auch mechanische Kraft sein, die eine Formänderung des medizinischen Hilfsmittels herbeiführt.

Ist die Zusammensetzung des dynamischen, synthetischen Materials oder alternativ der Anteil des zweiten Polymers und dessen Aufnahmekapazität für einen Stimulus bekannt, ist das Ausmaß der durch Änderung von Form oder Volumen erzeugten Gestaltveränderung des synthetischen Materials berechenbar. Gleiches gilt auch für SMA.

Solches thermoplastisches Material wird z. B. von der Firma Stratasys Inc. in Verbindung mit dem 4D Printing Project angeboten, zusammen mit Angaben dazu, welche Gestaltveränderung der Kontakt mit einem Stimulus oder mit verschiedenen Stimuli bewirkt. Stratasys Inc, setzt damit ein Druckverfahren in die Praxis um, das von Skylar Tibbits vom Self-Assembly Lab des Massachusetts Institute of Technology MIT, Boston, USA, entwickelt wurde und zu dem Autodesk Inc. die Software entwickelt hat, mit dem synthetisches Material, insbesondere dynamisches synthetisches Material mittels eines 3D Druckers zu einem Produkt aufgebaut werden kann, das dann unter Einwirkung eines bewegungsinduzierenden und damit gestaltverändernden Stimulus seine äußere Form in berechenbarer Weise ändert.

Die nachträgliche Verformbarkeit des dynamischen, synthetischen Materials oder einer SMA, die durch externe Stimuli induziert wird, kann genutzt werden, indem medizinische Hilfsmittel mindestens zum Teil aus SMP oder SMA hergestellt werden, die bei Einwirken eines Reizes in vorberechneter Weise ihre Gestalt ändern. Ist das medizinische Hilfsmittel eine Zahnkorrekturvorrichtung, so weist es eine Wand auf, die an der Oberfläche des bzw. der zu korrigierenden Zähne anliegt. Die Wand der Zahnkorrekturvorrichtung, die sich in berechneter Weise verformt, übt durch das Verformen Kraft auf den Zahn aus, an dem die Wand anliegt und bewegt ihn dadurch in die Soll-Stellung. In gleicher Weise kann das dynamische synthetische Material oder die SMA als Bestandteil eines Fixateurs, einer Orthese, eines Extenders oder eines Implantats eingesetzt sein, wenn diese medizinischen Hilfsmittel in einem Behandlungsplan eingesetzt werden, für den Voraussetzung ist, dass die medizinischen Hilfsmittel bei jedem Korrekturschritt eine Formänderung erfahren, um z. B. Knochen auszurichten oder zu verlängern oder um Wunden oder Narben zu behandeln, insbesondere zu dehnen, aber auch um Implantate an sich ändernde Bedingungen im Rahmen eines Behandlungsplans anzupassen. Der Vorteil des erfindungsgemäßen Verfahrens liegt darin, dass nicht für jeden Schritt ein neues medizinisches Hilfsmittel erstellt werden muss, und dass die Formänderung unter Berücksichtigung der körperlichen Entwicklung des Patienten in optimaler Weise erfolgt.

Die Information zur Wirkung des Stimulus, also der verschiedenen vorstehend genannten Parameter auf das jeweilige Material des medizinischen Hilfsmittels, werden jeweils vom Hersteller bekanntgegeben und sind somit beim Herstellen des medizinischen Hilfsmittels bekannt. Diese Information ist in der Steuereinheit hinterlegt und fließt in die Software bzw. den Algorithmus ein, der zur Berechnung des für den zweiten Korrekturschritt erforderlichen Stimulus eingesetzt wird.

Der berechnete Stimulus wird dann von der Steuereinheit ausgegeben und nachfolgend vorteilhaft an eine Behandlungsvorrichtung übermittelt. Die Behandlungsvorrichtung kann eine Vorrichtung sein, in die das medizinische Hilfsmittel eingesetzt wird oder die an das medizinische Hilfsmittel angelegt bzw. angesetzt oder angeschlossen wird. Die Behandlungsvorrichtung ist also z. B. als Behälter ausgebildet, der je nach Ausführung- temperiert werden kann und/oder mit Flüssigkeit gefüllt werden kann und/oder in den Strahlung, z. B. Infrarotstrahlung, eingebracht werden kann. Die Behandlungsvorrichtung kann auch einen Anschluss zur Verbindung mit dem medizinischen Hilfsmittel aufweisen und über die Verbindung einen Stimulus in das medizinische Hilfsmittel einleiten, insbesondere Strom oder Wärme. Die Behandlungsvorrichtung ist optional dazu ausgelegt, durch die Speichereinheit vorgegebene Teile des medizinischen Hilfsmittels einem Stimulus auszusetzen. Die Behandlungsvorrichtung kann auch zum Anwenden von mechanischer Kraft ausgelegt sein, um eine Formänderung des medizinischen Hilfsmittels herbeizuführen, z. B. durch Biegen oder Drücken einzelner Abschnitte des medizinischen Hilfsmittels. Die Behandlungsvorrichtung kann alternativ aber auch in das medizinische Hilfsmittel integriert sein, z. B. um Wärme als Stimulus einzusetzen. Bevorzugt ist die Behandlungsvorrichtung dazu ausgelegt, zwei oder mehr Stimuli in Kombination anzuwenden. Die Behandlungsvorrichtung zeichnet sich dadurch aus, dass sie den Stimulus nach der Vorgabe der Steuereinheit auslöst, der eine Formveränderung des medizinischen Hilfsmittels bewirkt. Der Stimulus wirkt meist für eine vorgegebene Zeitdauer auf das medizinische Hilfsmittel ein. Die Formänderung des medizinischen Hilfsmittels erfolgt also abschnittsweise bis eine maximale Formänderung des medizinischen Hilfsmittels erreicht ist. Dieser Vorgang der Formänderung kann also i. d. R. wiederholt werden, so oft wie das medizinische Hilfsmittel sich durch einen Stimulus weiter verformen lässt bzw. bis die endgültige Soll-Stellung des zu korrigierenden Körperteils erreicht ist.

Erfindungsgemäß weist die Steuereinheit einen Speicher (tracking system) für den zeitlichen Verlauf der Korrekturschritte, insbesondere für den Verlauf der Ist-Stellung des zu korrigierenden Körperteils auf.

Nach einer bevorzugten Weiterbildung des erfindungsgemäßen Verfahrens wird bei der Berechnung des Stimulus für den zweiten Korrekturschritt der zeitliche Verlauf, insbesondere der gesamte zeitliche Verlauf der Ist-Stellung des zu korrigierenden Körperteils von der Steuereinheit berücksichtigt.

Nach einer vorteilhaften Weiterbildung des erfindungsgemäßen Verfahrens gibt die Steuereinheit die Dauer des zweiten Korrekturschritts, die Größenordnung des nächsten Stimulus und/oder die zur Stimulierung ausgewählten bzw. berechneten Parameter über ein Ausgabe-Portal aus. Das Ausgabe-Portal kann gleich ausgebildet sein wie das Eingabe-Portal. Optional speichert die Speichereinheit diese Angaben und zieht sie ggf. für die Berechnung von Stimuli für spätere Korrekturschritte heran.

Nach einer Weiterbildung des erfindungsgemäßen Verfahrens werden die von der Steuereinheit ausgegebene Dauer des zweiten Korrekturschritts, die Größenordnung des nächsten Stimulus und/oder die zur Stimulierung ausgewählten Parameter manuell an eine Behandlungsvorrichtung übertragen. Alternativ übermittelt die Steuereinheit die ausgegebene Dauer des zweiten Korrekturschritts, die Größenordnung des nächsten Stimulus und/oder die zur Stimulierung ausgewählten Parameter automatisch an eine Behandlungsvorrichtung zur Durchführung des nächsten Korrekturschritts.

Weiter optional kann das Ausgabe-Portal Informationen zum Behandlungsfortschritt an den Patienten übermitteln, z. B. kann der Fortschritt der Behandlung angezeigt werden oder es können Warnhinweise ausgegeben werden, z. B. wenn das medizinische Hilfsmittel nicht ausreichend lang getragen wird. Zudem können auch Hinweise auf die Zeit bis zum nächsten Korrekturschritt angezeigt werden. Des Weiteren kann ein Hinweis an den Patienten angezeigt werden, in welcher Position das medizinische Hilfsmittel in die Behandlungsvorrichtung einzusetzen ist (z. B. längs/quer oder senkrecht/waagerecht) bzw. in welcher Position die Behandlungsvorrichtung an das medizinische Hilfsmittel anzubringen ist. Das Ausgabe-Portal kann in Verbindung mit dem Eingabe-Portal auch eine Chat-Funktion unterstützen, die eine direkte Kommunikation mit dem Arzt oder Hersteller ermöglicht.

Das erfindungsgemäße Verfahren umfasst bevorzugt einen eigenen Datensatz für jedes medizinische Hilfsmittel, der nach Eingeben einer Bearbeitungsberechtigung in dem Eingabe-Portal bearbeitet werden kann, z. B. durch Eingeben einer neuen Ist-Stellung des zu korrigierenden Körperteils. Auf diese Weise ist gesichert, dass jeweils Daten zu dem zugehörigen medizinischen Hilfsmittel, z. B. zu einer Zahnkorrekturvorrichtung, einem Fixateur, einem Extender oder einer Orthese eingegeben werden.

Die Erfindung umfasst weiter eine Steuereinheit, die Mittel zum Berechnen eines Stimulus und einen Speicher für Daten aufweist. Die Mittel zum Berechnen sind dazu ausgelegt, den Stimulus für den nächsten Korrekturschritt zu berechnen und ggf. auszuwählen, wobei Daten zur Wirkung mindestens eines Stimulus, bevorzugt jedoch mehrerer Stimuli und Daten zur Soll-Stellung des zu korrigierenden Körperteils für die Ausgangsstellung und für jeden einzelnen Korrekturschritt im Speicher enthalten sind und wobei Daten zur Ist-Stellung des zu korrigierenden Körperteils für die Berechnung des Stimulus vorab über ein mit der Steuereinheit in Verbindung stehendes Eingabe-Portal eingegeben werden. Die Mittel zum Berechnen weisen eine Software auf, die aus der Soll-Stellung und der Ist-Stellung des zu korrigierenden Körperteils nach einem ersten Korrekturschritt sowie ggf. einer Abweichung zwischen der Soll- und der aktuellen Ist-Stellung und der Wirkung des jeweiligen mindestens einen Stimulus auf das medizinische Hilfsmittel den für den zweiten Korrekturschritt berechnet. Dabei kann die Software den Stimulus oder eine Kombination von Stimuli für den zweiten Korrekturschritt wechseln oder wählen, in Abhängigkeit vom Ausmaß der im zweiten Korrekturschritt zu bewirkenden Formänderung des medizinischen Hilfsmittels zur Bewegung des zu korrigierenden Körperteils. Außerdem kann die Software die Intensität des Stimulus ermitteln, also z. B. die Höhe der Temperatur, die Dauer, über die der Stimulus einwirken muss. Der Stimulus kann in Abhängigkeit von einer gewählten Behandlungsvorrichtung gewählt werden, so dass sichergestellt ist, dass die Behandlungsvorrichtung den berechneten Stimulus auch auf das medizinische Hilfsmittel anwenden kann.

Der berechnete Stimulus für den zweiten Korrekturschritt wird dann ausgegeben. Entweder wird der Stimulus ausgedruckt oder auf einem Ausgabe-Portal, z. B. einem Bildschirm, angezeigt. Alternativ wird der berechnete Stimulus an eine Behandlungsvorrichtung übermittelt und dort umgesetzt, d. h., der Stimulus wirkt auf das medizinische Hilfsmittel ein.

Das Eingeben der aktuellen Ist-Stellung des zu korrigierenden Körperteils erfolgt wie vorstehend beschrieben über ein Eingabe-Portal. Vorteilhaft ist das Eingabe-Portal mit der Steuereinheit verbunden, weiter vorteilhaft dient das Eingabe-Portal auch als Ausgabe-Portal.

Es wird weiter bevorzugt, wenn das Eingabe-Portal nur mit einer Zugangsberechtigung für eine Eingabe geöffnet werden kann. Es wird weiter bevorzugt, wenn eine Eingabe für eine spezifische Korrekturvorrichtung nur erfolgen kann, wenn der spezifische Datensatz für diese Korrekturvorrichtung mittels eines Codes geöffnet wird. Dadurch wird gewährleistet, dass die anstehende Formänderung des medizinischen Hilfsmittels korrekt erfolgt, weil irrtümliche Fehlbedienungen ausgeschossen sind, nachdem der Zugangscode eingegeben wurde.

Es wird weiter bevorzugt, dass die Steuereinheit und ggf. die Behandlungsvorrichtung für das medizinische Hilfsmittel so voreingestellt sind, dass bei Fehlen oder Ausfall von Teilen der Steuereinheit bzw. der Software die Aktivierung des medizinischen Hilfsmittels durch den Stimulus für einen folgenden Korrekturschritt nach dem ursprünglichen Behandlungsplan erfolgt, so dass die Behandlung nicht unterbrochen wird, sondern lediglich weniger optimiert fortgesetzt werden kann.

Details der Erfindung werden nachfolgend an einem Ausführungsbeispiel erläutert. Es zeigen:
- Fig. 1: Ablaufschema zur schematischen Zuordnung von Eingabe-Portal und Behandlungsvorrichtung
- Fig. 2a: Ablaufschema zur vollständigen Berechnung des Stimulus
- Fig. 2b: Ablaufschema zur Berechnung der Differenz zwischen Ist-Stellung und Soll-Stellung des zu korrigierenden Zahns
- Fig. 3: Durchführen der Formänderung

Das nachfolgende Ausführungsbeispiel bezieht sich auf einen zu korrigierenden Zahn. Es lässt sich aber ohne Weiteres auch auf einen zu korrigierenden Knochen (Richten eines gebrochenen Knochens, Verlängerung eines Knochens) übertragen oder auf das Korrigieren einer Narbe bzw. einer Wunde. In einem ersten Schritt des erfindungsgemäßen Verfahrens zur Berechnung eines Stimulus zum Auslösen einer Verformung eines medizinischen Hilfsmittels wird die Ist-Stellung des zu korrigierenden Zahns erfasst und es wird die endgültige Soll-Stellung des zu korrigierenden Zahns erfasst. Es wird ein Behandlungsplan erstellt, bei dem der Weg von der aktuellen Ist-Stellung zur endgültigen Soll-Stellung in mindestens zwei Korrekturschritte, oft auch mehrere Korrekturschritte aufgeteilt wird. Korrespondierend zum Behandlungsplan wird die Zahnkorrekturvorrichtung, im Folgenden kurz "Korrekturvorrichtung" erstellt. Das Material des medizinischen Hilfsmittels ändert unter dem Einfluss eines Stimulus in vorberechneter Weise seine Form, bevorzugt von einer Ist-Stellung in eine Soll-Stellung. Art und Intensität des Stimulus beeinflussen das Ausmaß der Formänderung.

In einer Steuereinheit werden die erforderlichen Daten der Ist- und der endgültigen Soll-Stellung des zu korrigierenden Zahns, die Soll-Stellungen nach den einzelnen Korrekturschritten und die Art und Intensität mindestens eines Stimulus, bevorzugt mehrerer Stimuli hinterlegt. Die Steuereinheit weist hierfür einen Speicher auf. Die Steuereinheit ist weiter mit einem Prozessor und mit einer Software ausgestattet, die nach Abschluss eines ersten Korrekturschritts einen Stimulus zum Auslösen einer Formänderung des medizinischen Hilfsmittels für den zweiten bzw. nächsten folgenden Korrekturschritt berechnet.

Die Steuereinheit kann eine eigene Vorrichtung mit einem Eingabe-Portal, z. B. einer Tastatur oder einer Touch-Screen-Maske, und vorteilhaft mit einem Ausgabe-Portal, z. B. einer Bildschirmanzeige oder einem Drucker, sein.

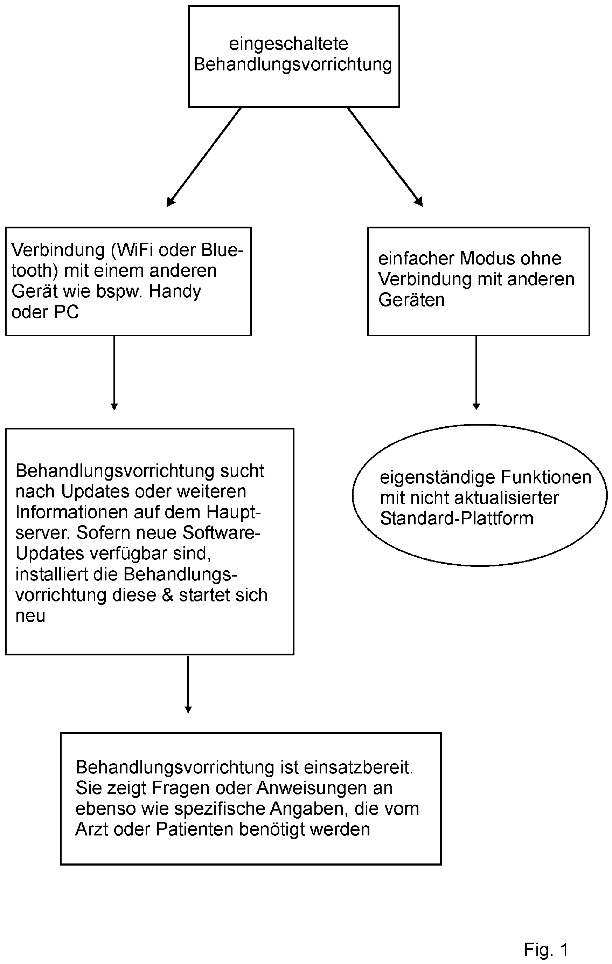

Bevorzugt steht die Steuereinheit -wie in Fig. 1 dargestellt- in Verbindung mit einem Rechner, z. B. in Gestalt eines Smartphones, eines Tablets oder eines Desktop-Rechners. Um Verbindung mit der Steuereinheit aufzunehmen, kann eine Verbindung zum Eingabe-Portal z. B. als eigene Anwendung (App) heruntergeladen werden, deren Eingabe-Oberfläche mit dem Eingabe-Portal in Verbindung steht und deren Ausgabe-Oberfläche mit dem Ausgabe-Portal der Steuereinheit in Verbindung steht. Die Tastatur des Rechners und der Bildschirm des Rechners können für Eingaben in die Eingabe-Oberfläche und damit in das Eingabe-Portal genutzt werden. Das Verbinden der Steuereinheit mit einem Rechner hat den Vorteil, dass der Speicher und die Software der Steuereinheit stets durch Updates aktuell gehalten werden können. Alternativ kann eine Stand-Alone Steuereinheit verwendet werden.

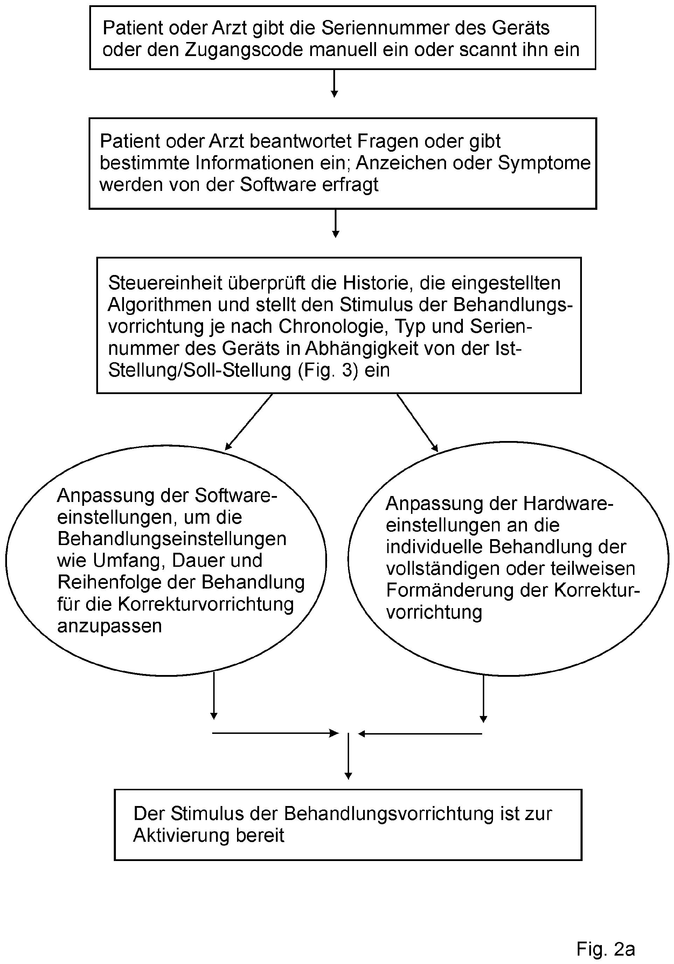

Ausgehend von einer mit einem Rechner verbundenen Steuereinheit ist diese Steuereinheit nach dem Einschalten und dem Laden eventuell vorhandener Updates für eine Dateneingabe bereit, entweder für das Anlegen einer neuen Datei für eine neue Korrekturvorrichtung oder für die Auswertung eines erfolgten Korrekturschritts und der Berechnung eines Stimulus für einen folgenden Korrekturschritt. Die Steuereinheit bzw. das Eingabe-Portal können zur Benutzung für den Patienten, den behandelnden Arzt oder den Hersteller des medizinischen Hilfsmittels ausgelegt sein, je nachdem, wer eine neue Datei für ein medizinisches Hilfsmittel anlegt oder einen Korrekturschritt auswertet und den Stimulus für einen weiteren Korrekturschritt berechnet. Vorteilhaft ist gemäß Fig. 2a eine Zugangsberechtigung für den jeweiligen Benutzer der Steuereinheit vorgesehen, um sicherzustellen, dass nur Berechtigte und ggf. in die Benutzung der Steuereinheit eingewiesene Benutzer Eingaben vornehmen. Es wird ebenfalls empfohlen, für jede Korrekturvorrichtung eine eigene Datei mit eigenem Zugangscode anzulegen, um sicherzustellen, dass ein Benutzer, der z. B. als behandelnder Arzt mehrere Korrekturvorrichtungen betreut, Eingaben stets für die richtige Korrekturvorrichtung vornimmt.

Am Ende eines ersten Korrekturschritts erfolgt die Auswertung der bisher erreichten Formänderung des bzw. der zu korrigierenden Zähne. Die aktuell erreichte Ist-Stellung des zu korrigierenden Zahns wird erfasst, entweder manuell z. B. durch einen Abdruck und Vermessen des Abdrucks, oder digital durch Vermessen und Bestimmen der aktuellen Position des zu korrigierenden Zahns. Die so erhaltenen Daten werden in das Eingabe-Portal der Steuereinheit eingegeben, entweder über eine Tastatur oder mittels Datenübertragung, z. B. per Kabel, Bluetooth oder eine kabellose Netzwerkverbindung (WLAN, WiFi). Optional stellt das Eingabe-Portal noch Fragen an den Patienten, z. B. zur Verträglichkeit des medizinischen Hilfsmittels oder der Intensität der Korrektur. Vorteilhaft sind standardisierte Antworten (z. B.: gut - mittel - schlecht; hoch - mittel - niedrig) vorgesehen, die in einem Algorithmus verarbeitet werden können, der den ersten Korrekturschritt auswertet.

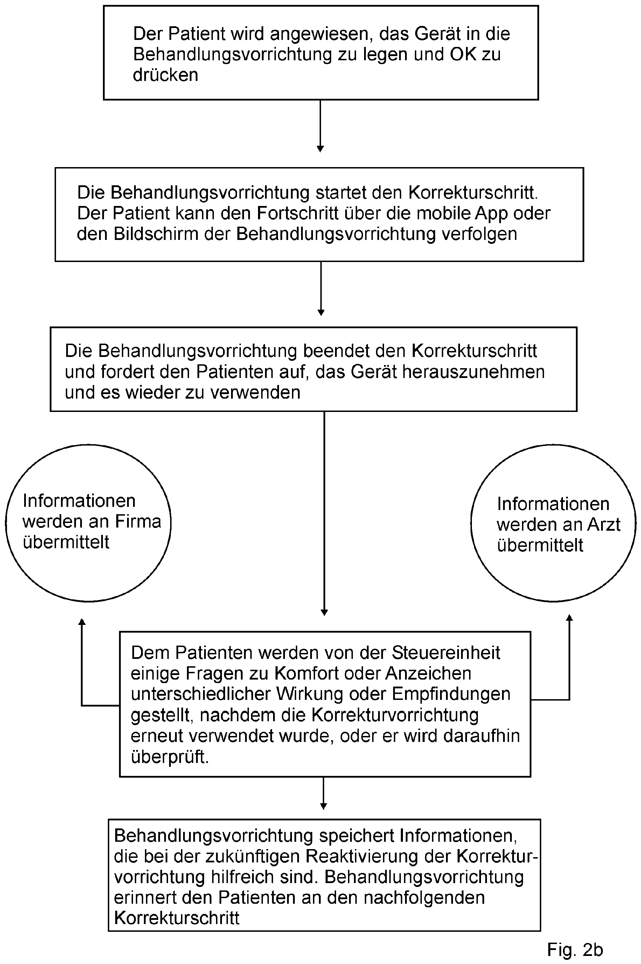

Zum Berechnen des Stimulus, der die Formänderung des medizinischen Hilfsmittels für den nachfolgenden Korrekturschritt bewirken soll, wird gemäß Fig. 2b ermittelt, ob eine Abweichung zwischen der aktuellen Ist-Stellung und der gemäß Behandlungsplan vorgegebenen bzw. erwarteten Soll-Stellung des zu korrigierenden Zahns vorliegt. Eine solche Abweichung wird z. B. mittels Vermessen der Zahnstellung durch einen Intraoral-Scanner oder durch einen Vergleich digitaler Fotografien ermittelt; sie kann aber auch durch das Erstellen eines aktuellen Abdrucks und Vergleich mit dem vorangegangenen Abdruck, der vor Beginn des abgelaufenen Korrekturschritts abgenommen wurde, erfolgen.

Bei Vorliegen einer solchen Abweichung wird diese für die Berechnung des Stimulus berücksichtigt. Des Weiteren werden gemäß Fig. 2a die Antworten auf die Fragen der Steuereinheit (soweit solche Fragen gestellt wurden) für die Berechnung des Stimulus berücksichtigt. Die Steuereinheit bzw. der Algorithmus berücksichtigt weiter die im ursprünglichen Behandlungsplan vorgegebenen Einstellungen für den Stimulus, der zu Beginn des nachfolgenden Korrekturschritts auf das medizinische Hilfsmittel einwirken soll. Die vorgegebenen Einstellungen werden nun unter Berücksichtigung eventueller Abweichungen der Ist-Stellung von der Soll-Stellung sowie der Antworten der Patienten z. B. zur Verträglichkeit oder Intensität der Behandlung neu berechnet, um mit minimaler Belastung des Patienten schnellstmöglich den zu korrigierenden Zahn in die endgültige Soll-Stellung zu bewegen. Ggf. wählt die Steuereinheit bzw. der Algorithmus, je nach dem in welchem Umfang eine Formänderung des medizinischen Hilfsmittels erforderlich ist, einen anderen Stimulus und/oder eine andere Intensität, z. B. wird von Wärme zu Flüssigkeit gewechselt oder zu einer Kombination von Stimuli, z. B. Wärme und Flüssigkeit. Das erfindungsgemäße Verfahren gewährleistet damit, dass das Korrigieren der Zahnstellung nicht nach einem starren, zu Beginn der Behandlung vorgegebenen Behandlungsplan erfolgt, sondern dass die Korrektur optimiert unter Berücksichtigung des tatsächlichen Verlaufs der Korrektur durchgeführt wird. Dies resultiert in einer kurzen und für den Patienten schonenden Korrekturbehandlung.

Die neue Berechnung des Stimulus für die Formänderung des medizinischen Hilfsmittels wird dann von der Steuereinheit über das Ausgabe-Portal abgegeben. Nach einer ersten Alternative wird die Berechnung des Stimulus vom Ausgabe-Portal z. B. ausgedruckt oder angezeigt und wird von Hand an eine Behandlungsvorrichtung übertragen. Die Behandlungsvorrichtung kann unmittelbar an das medizinische Hilfsmittel angebracht sein, z. B. als Wärmequelle. Oft ist die Behandlungsvorrichtung als Behälter ausgebildet, in dem das medizinische Hilfsmittel aufgenommen werden kann. Die Behandlungsvorrichtung ist weiter dazu ausgelegt, mindestens einen Stimulus auf das medizinische Hilfsmittel einwirken zu lassen, vorteilhaft können zwei oder mehr Stimuli auf das medizinische Hilfsmittel einwirken, z. B. Temperatur, Flüssigkeit und Strahlung, jeweils einzeln oder in Kombination.

Nach einer alternativen Ausführung ist die Steuereinheit mit der Behandlungsvorrichtung verbunden und steuert diese. Die Verbindung kann durch Einbau in das Gehäuse der Behandlungsvorrichtung erfolgen, sie kann aber auch durch eine kabelgebundene oder drahtlose Verbindung erfolgen, indem die Steuereinheit Betätigungssignale an die Behandlungsvorrichtung sendet.

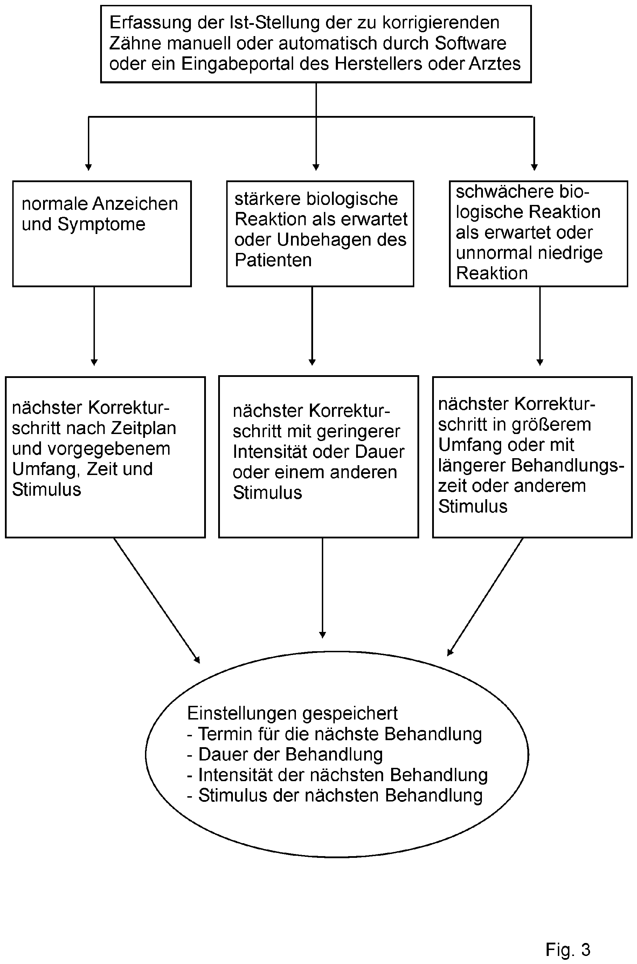

Die neue Berechnung löst beim Steuern der Behandlungsvorrichtung gemäß Fig. 3 die Aktivierung eines oder mehrerer Stimuli nach den Vorgaben der vorangegangenen Berechnung der Steuereinheit aus. Ggf. veranlasst die Steuereinheit vorab noch die Anzeige von Hinweisen über das Ausgabe-Portal, z. B. den Hinweis, das medizinische Hilfsmittel in die Behandlungseinrichtung einzulegen, oder den Hinweis, nach dem Einlegen des medizinischen Hilfsmittels die Behandlungseinrichtung einzuschalten und damit die Formänderung infolge des Einwirkens des Stimulus bzw. der kombinierten Stimuli auszulösen. Weiter optional zeigt das Ausgabe-Portal den Verlauf des Einwirkens des Stimulus und die daraus resultierende Formänderung des medizinischen Hilfsmittels an.

Nach Abschluss des Einwirkens des Stimulus zeigt das Ausgabe-Portal optional Informationen über den nachfolgenden Korrekturschritt an, z. B. wie lange der nachfolgende Korrekturschritt dauert, wie lange das medizinische Hilfsmittel jeweils zu tragen ist, um einen optimalen Behandlungserfolg zu gewährleisten.

Optional zeigt das Ausgabe-Portal nach dem Einsetzen des medizinischen Hilfsmittels durch den Patienten noch einige Fragen an den Patienten, die insbesondere den Sitz der in ihrer Form geänderten Korrekturvorrichtung im Mund betreffen sowie Fragen zu den (Miss-)empfindungen während des Tragens des medizinischen Hilfsmittels oder Fragen zur Auswirkung des medizinischen Hilfsmittels auf die zu korrigierenden oder andere Zähne. Auf diese Weise wird sichergestellt, dass eventuelle Fehl-Verformungen des medizinischen Hilfsmittels frühzeitig entdeckt werden.

Die Steuereinheit speichert, wie in Fig. 3 gezeigt, die zur Berechnung des Stimulus eingesetzten Daten, so dass am Ende der Behandlung sämtliche Informationen zur Behandlung verfügbar sind. Ggf. werden diese Informationen an den behandelnden Arzt und/oder den Hersteller des medizinischen Hilfsmittels übermittelt. Umfasst die Behandlung mit der Zahnkorrekturvorrichtung mehr als zwei Korrekturschritte, können zur Berechnung des Stimulus für einen nachfolgenden Korrekturschritt sämtliche aus vorangegangenen Korrekturschritten verfügbare Informationen eingesetzt werden.

Abschließend wird noch kurz erläutert, in welcher Weise die gemäß Fig 2b ermittelten Unterschiede zwischen der Ist-Stellung des zu korrigierenden Zahns und der Soll-Stellung des zu korrigierenden Zahns in die Berechnung des Stimulus für die Formveränderung des medizinischen Hilfsmittels für den jeweils nächsten Korrekturschritt einfließt. Wird nach Abschluss eines ersten Korrekturschrittes festgestellt, dass keine Abweichung zwischen Ist-Stellung und Soll-Stellung vorliegt, erfolgt die Berechnung des Stimulus zur Formänderung des medizinischen Hilfsmittels für den nachfolgenden Korrekturschritt gemäß dem ursprünglichen Behandlungsplan.

Hat sich der zu korrigierende Zahn weiter bewegt als erwartet oder meldet der Patient Beschwerden, dann wird der Behandlungsplan für den nächsten Korrekturschritt geändert. Von der Steuereinheit bzw. durch den Algorithmus wird ein weniger intensiver Stimulus oder ein anderer, weniger intensiver Stimulus ausgewählt (z. B. Flüssigkeit an Stelle von Strahlung).

Hat sich der zu korrigierende Zahn weniger bewegt als erwartet oder meldet der Patient ausdrücklich gutes Befinden, dann wird der Behandlungsplan für den nächsten Korrekturschritt geändert. Von der Steuereinheit bzw. durch den Algorithmus wird ein intensiverer Stimulus oder ein anderer, intensiverer Stimulus gewählt (z. B. ein längeres Einwirken des Stimulus). Es kann auch jeweils eine Kombination von Stimuli eingesetzt werden, stets im Rahmen der Stimuli, die die jeweilige Behandlungsvorrichtung auf das medizinische Hilfsmittel einwirken lassen kann.

Nachfolgend wird erläutert, wie die Steuereinheit bzw. die Software erstellt wird, die zur Umsetzung des erfindungsgemäßen Verfahrens benötigt werden. Es können z. B. ATMEL-Chips mit einem Zugangscode versehen werden, die den Zugang zur Steuereinheit auf Berechtigte begrenzen, da der Chip ohne den korrekten Steuercode nicht mehr zu öffnen ist. Der Zugangscode kann z. B. mit AVR Studio Software erstellt werden. Als Steuereinheit kann z. B. auch ein Espressif Systems Wi-Fi/BLESOC Mikrochip (z. B. esp8266 WIFI/esp32 WIFI/BLE) eingesetzt werden, der auf einem 32 bit Mikrocontroller basiert, der eine ausreichende Anzahl von digitalen I/O Zugängen aufweist, um alle elektronischen Peripheriegeräte und analogen Eingänge zur Erfassung von Sensorsignalen zu steuern und der auch ausreichend Speicherkapazität aufweist, um die Softwarecodes zu speichern.

Zum Erstellen der Software bzw. des Algorithmus kann eine Arduino IDE-Plattform eingesetzt werden, die zum Erstellen interaktiver Software geeignet ist. Die Software wird dann mittels eines geeigneten Druckers auf einen Chip übertragen. Später können in Serie hergestellte gedruckte Leiterplatten (PCB printed circuit boards) eingesetzt werden, auf denen die Software gespeichert wird und die als Steuereinheit dienen. Ist die Steuereinheit in einer Behandlungsvorrichtung eingebaut, kann sich diese vorteilhaft selbstständig mit einem Server, z. B. des Herstellers, verbinden und Updates herunterladen. Es wird bevorzugt, wenn eine Eingabemaske, die mit dem Eingabe-Portal in Verbindung steht, und ggf. eine Anzeige über den Fortschritt bzw. Status der Formänderung als Anwendung (App) auf externe Rechner wie Smartphones, Tablets oder Desktop-Computer heruntergeladen werden können.

Der Chip bzw. die Leiterplatte, auf denen die Software gespeichert ist, kann nun zahlreiche Vorgänge steuern, darunter WiFi- oder Bluetooth-Verbindungen zur Übertragung von Daten von Rechnern wie z. B. Smartphones, Tablets oder Desktop-Computern, das Eingabe- und das Ausgabe-Portal, die ggf. auf diesen Rechnern angezeigt werden, die Verwaltung von Speichern, auf denen Daten zum Patienten und/oder zur Korrekturvorrichtung bzw. zum Behandlungsplan gespeichert sind und ggf. auch die Behandlungsvorrichtung, durch die ein Stimulus auf das medizinische Hilfsmittel einwirkt, um einen folgenden Korrekturschritt vorzubereiten. Der Chip bzw. die Leiterplatte steuert in diesem Fall z. B. das Ein- und Ausschalten des jeweiligen Stimulus, z. B. einer Heizung, einer Kühlung, einer Strahlungsquelle oder der Zugabe von Flüssigkeit, sowohl einzeln als auch in Kombination. So kann z. B. eine Formänderung eines medizinischen Hilfsmittels erfordern, dass zunächst eine vorgegebene Menge an Flüssigkeit, z.B. Wasser, in einen Behälter eingelassen wird, der das medizinische Hilfsmittel enthält. Zuvor ist anzuzeigen, dass das medizinische Hilfsmittel richtig in den Behälter eingesetzt wurde. Dies kann optisch durch eine Kamera oder durch Sensoren oder Schalter an den Chip oder die Leitplatte gemeldet werden. Anschließend muss das Wasser zunächst erwärmt und anschließend gekühlt werden, wobei Zeitvorgaben für das Erwärmen und das Abkühlen einzuhalten sind. Schließlich ist anzuzeigen, dass die Formänderung abgeschlossen ist. Anschließend sind optional die Daten der Formänderung, sowohl die Angaben zur Ist- und Soll-Stellung als auch die Eingaben des Patienten und die Berechnung des Stimulus, zu speichern und ggf. an Arzt und Hersteller des medizinischen Hilfsmittels zu übermitteln.

Die Steuereinheit bzw. der Chip oder die Leiterplatte kann mit einem Server des Arztes oder Herstellers in Verbindung gebracht werden, der über eine stationäre IP-Adresse mit einem Domain-Namen erreichbar ist. Vom Server kann die Steuereinheit Updates abrufen oder auch Modifikationen der Behandlungsvorrichtung. Es kann z. B. ein raspberry pi-Server eingesetzt werden, auf dem das Betriebssystem raspbian, ein apache-Server und in php programmierte Inhalte für Web-Anwendungen installiert sind.

In einem zweiten Ausführungsbeispiel erläutern wir eine mögliche Ausführung des erfindungsgemäßen Verfahrens unter Nutzung der erfindungsgemäßen Steuereinheit. Ein Patient trägt eine erste Korrekturvorrichtung für eine Woche. Laut Behandlungsplan soll das medizinische Hilfsmittel jeweils nach einer Woche zwei Formänderungen durchlaufen. Die erfindungsgemäße Steuereinheit bzw. Software ist nach Eingeben des Zugangscodes dazu ausgelegt, den Patienten zunächst zu fragen, welche Korrekturvorrichtung er trägt und seit wann er das medizinische Hilfsmittel trägt. Nach dem Eingeben weiterer Daten zur Ist-Stellung des zu korrigierenden Zahns und optional zu Patientendaten wird der für die anstehende Formänderung des medizinischen Hilfsmittels erforderliche Stimulus berechnet. Dabei wird berücksichtigt, dass der Stimulus für den zweiten Korrekturschritt, also am Beginn der zweiten Woche zu errechnen ist. Es wird also fehlerfrei der korrekte Stimulus berechnet und die Behandlungsvorrichtung wird entsprechend durch die Steuereinheit gesteuert. Nach der zweiten Woche erfolgt derselbe Ablauf unter Berücksichtigung des aktualisierten Zeitablaufs. Die Berechnung des Stimulus erfolgt für den dritten Korrekturschritt, also für die dritte Woche. Versucht der Patient irrtümlich, zwischen den einzelnen Korrekturschritten oder nach Ablauf der Korrekturbehandlung eine Formänderung des medizinischen Hilfsmittels auszulösen, ist gemäß der vorliegenden Ausführung der Erfindung vorgesehen, dass die Steuereinheit anzeigt, dass zur Zeit keine Formänderung vorgesehen ist, oder dass die Form des medizinischen Hilfsmittels aktuell ist. Auf diese Weise wird verhindert, dass irrtümlich unerwünschte Formänderungen an dem medizinischen Hilfsmittel vorgenommen werden.

Nach einem weiteren Ausführungsbeispiel wird eine ein- oder mehrteilige Orthese oder ein Kunststoffverband wie vorstehend beschrieben mittels 3D-Druck aus einem dynamischen synthetischen Material oder abschnittsweise aus einem dynamischen synthetischen Material in Verbindung mit einem statischen synthetischen Material hergestellt. Orthese oder Kunststoffverband sind zur Korrektur der Knochenstellung individuell an einen Patienten angepasst. Die Knochenstellung, z. B. die Stellung der Fußknochen, soll nach einem mehrschrittigen Behandlungsplan korrigiert werden, z. B. um das Fußgewölbe oder die Stellung der Zehen wiederherzustellen.

Es wird ein Behandlungsplan erstellt, der mehrere Korrekturschritte vorsieht. Am Ende des ersten Behandlungsplans wird die Knochenstellung erfasst, z. B. durch Röntgen oder andere bildgebende Verfahren oder durch Vermessen des Fußes und der Knochenstellung. Bei der Berechnung des nächstfolgenden Korrekturschritts wird die Ist-Stellung des bzw. der zu korrigierenden Fußknochen erfasst und bei der Berechnung des Stimulus berücksichtigt, der erforderlich ist, um die Orthese oder den Kunststoffverband zur Durchführung des nächsten Korrekturschritts zu verformen. Auch bei diesem Ausführungsbeispiel können natürlich weitere der vorstehend genannten, individuellen, auf den Patienten bezogenen Parameter in die Berechnung des Stimulus einfließen.

Ein abschließendes Ausführungsbeispiel bezieht sich auf die Versorgung von Wunden, die mit Hilfe von medizinischen Hilfsmitteln erfolgt. Das medizinische Hilfsmittel ist hier z. B. als Klammer oder als Nahtmaterial ausgebildet. Die nach einem mehrschrittigen Behandlungsplan erfolgenden Formänderungen der Klammer oder des Nahtmaterials helfen, Wunden so zu schließen, dass physiologische Schäden minimiert werden und gleichzeitig der ästhetische Aspekt einer Narbe optimiert wird. Bei der Verformung der Klammer oder des Nahtmaterials durch einen Stimulus nach dem vorstehend beschriebenen, erfindungsgemäßen Verfahren müssen Klammern und Nahtmaterial nicht stets neu gesetzt werden und können durch erfindungsgemäß berechnete Stimuli inkrementell in kleinsten Schritten in ihrer Form verändert und damit optimal an die medizinischen Erfordernisse angepasst werden. Das Nahtmaterial bzw. die Klammern können z. B. durch Wärme oder durch Strahlung, aber auch durch Strom in ihrer Form verändert werden.

## Patentansprüche

1. Verfahren zum Berechnen eines Stimulus für die Formänderung eines medizinischen Hilfsmittels, um die Form des Hilfsmittels von einer Ist-Stellung bis zu einem endgültigen Soll-Zustand zu ändern, mit den Schritten:
- Erfassen der Ist-Stellung des zu korrigierenden Körperteils nach einem ersten Korrekturschritt in einer Steuereinheit,
- Vergleichen der Ist-Stellung des zu korrigierenden Körperteils mit einer Soll-Stellung, die in der Steuereinheit gespeichert ist,
**dadurch gekennzeichnet, dass** das Verfahren weiter die Schritte aufweist:
- ggf. Erfassen der Abweichung von Ist- und Soll-Stellung des zu korrigierenden Körperteils durch die Steuereinheit,
- Berechnen des Stimulus, der auf das medizinische Hilfsmittel zum Ausführen eines zweiten Korrekturschritts einzuwirken hat, unter Berücksichtigung der Abweichung von Ist- und Soll-Zustand des zu korrigierenden Körperteils durch die Steuereinheit, wobei in der Steuereinheit die Wirkung des Stimulus auf das medizinische Hilfsmittel hinterlegt ist.

2. Verfahren nach Anspruch 1, wobei der Stimulus ausgewählt ist aus einer Gruppe, die die Parameter Zeit, Temperatur, pH-Wert, Strom Strahlung, Schall, Belichtung, oder Flüssigkeit umfasst oder wobei der Stimulus ausgewählt ist aus einer Kombination dieser Parameter.

3. Verfahren nach Anspruch 1 oder 2, wobei die Ist-Stellung des zu korrigierenden Körperteils erfasst wird durch einen Abdruck, durch digitales Vermessen der Stellung des Körperteils oder durch Signale eines Sensors, der die Stellung des Körperteils erfasst.

4. Verfahren nach einem der vorstehenden Ansprüche, wobei die Ist-Stellung des zu korrigierenden Körperteils durch manuelle Eingabe in die Steuereinheit oder durch ein Eingabe-Portal des Herstellers des medizinischen Hilfsmittels oder durch ein Eingabe-Portal des behandelnden Arztes erfolgt.

5. Verfahren nach einem der vorstehenden Ansprüche, wobei die Steuereinheit mit einer Behandlungsvorrichtung in Verbindung steht, die dazu ausgelegt ist, den berechneten Stimulus auf das medizinische Hilfsmittel einwirken zu lassen.

6. Verfahren nach einem der vorstehenden Ansprüche, wobei die Steuereinheit den zeitlichen Verlauf des Ist-Zustandes des zu korrigierenden Körperteils speichert.

7. Verfahren nach einem der vorstehenden Ansprüche, wobei die Steuereinheit den zeitlichen Verlauf der Ist-Zustände des zu korrigierenden Körperteils bei der Berechnung des Stimulus für den zweiten Korrekturschritt berücksichtigt.

8. Verfahren nach einem der vorstehenden Ansprüche, wobei die Steuereinheit die Dauer des zweiten Korrekturschritts, die Größenordnung des nächsten Stimulus und/oder die zur Stimulierung ausgewählten Parameter berechnet, ausgibt und speichert.

9. Verfahren nach einem der vorstehenden Ansprüche, wobei die von der Steuereinheit ausgegebene Dauer des zweiten Korrekturschritts, die Größenordnung des nächsten Stimulus und/oder die zur Stimulierung ausgewählten Parameter manuell an eine Behandlungsvorrichtung übertragen werden oder wobei die Steuereinheit die ausgegebene Dauer des zweiten Korrekturschritts, die Größenordnung des nächsten Stimulus und/oder die zur Stimulierung ausgewählten Parameter automatisch zur Steuerung einer Behandlungsvorrichtung einsetzt.

10. Verfahren nach einem der vorstehenden Ansprüche, wobei für jedes medizinische Hilfsmittel ein eigener Datensatz angelegt wird, der nach Eingeben einer Bearbeitungsberechtigung bearbeitet werden kann.

11. Steuereinheit zur Durchführung eines Verfahrens nach Anspruch 1, verbunden mit einem Eingabeportal für das Eingeben des Ist-Zustands des zu korrigierenden Körperteils und mit Mitteln zum Berechnen eines Stimulus für die Formänderung eines medizinischen Hilfsmittels sowie mit einem Speicher, wobei die Wirkung des Stimulus auf das medizinisches Hilfsmittel und die Sollstellung des zu korrigierenden Körperteils im Speicher hinterlegt sind, **dadurch gekennzeichnet, dass** der Speicher der Steuereinheit dazu ausgelegt ist, den zeitlichen Verlauf von Korrekturschritten zu speichern.

12. Steuereinheit nach Anspruch 11, wobei Steuereinheit und Eingabeportal räumlich getrennt sind, und wobei die Daten vom Eingabeportal zur Steuereinheit übertragen werden.

13. Steuereinheit nach Anspruch 11 oder 12, wobei das Eingabeportal mittels einer Zugangsberechtigung geöffnet wird.

## Claims

1. A method for calculating a stimulus for the change of shape of a medical aid, in order to change the shape of the aid from an actual position to a final nominal state, comprising the following steps:
- capturing the actual position of the body part to be corrected after a first correction step in a control unit,
- comparing the actual position of the body part to be corrected with a nominal position which is saved in the control unit,
**characterized in that** the method further has the following steps:
- optionally, capturing the deviation between the actual position and nominal position of the body part to be corrected by the control unit,
- calculating the stimulus which has to act on the medical aid in order to execute a second correction step, taking account of the deviation between the actual state and the nominal state of the body part to be corrected, by the control unit, wherein the effect of the stimulus on the medical aid is stored in the control unit.

2. The method according to Claim 1, wherein the stimulus is selected from a group which comprises the parameters of time, temperature, pH value, current, radiation, sound, light exposure, or liquid, or wherein the stimulus is selected from a combination of these parameters.

3. The method according to Claim 1 or 2, wherein the actual position of the body part to be corrected is captured by an impression, by digital measurement of the position of the body part or by signals of a sensor which captures the position of the body part.

4. The method according to one of the preceding claims, wherein the actual position of the body part to be corrected is effected by a manual input into the control unit or by an input portal of the manufacturer of the medical aid or by an input portal of the treating physician.

5. The method according to one of the preceding claims, wherein the control unit is connected to a treatment device which is designed to allow the calculated stimulus to act on the medical aid.

6. The method according to one of the preceding claims, wherein the control unit saves the temporal course of the actual state of the body part to be corrected.

7. The method according to one of the preceding claims, wherein the control unit takes account of the temporal course of the actual states of the body part to be corrected when the stimulus for the second correction step is calculated.

8. The method according to one of the preceding claims, wherein the control unit calculates, outputs and saves the duration of the second correction step, the magnitude of the next stimulus and/or the parameters selected for stimulation.

9. The method according to one of the preceding claims, wherein the duration of the second correction step output by the control unit, the magnitude of the next stimulus and/or the parameters selected for stimulation are transmitted manually to a treatment device, or wherein the control unit automatically utilizes the output duration of the second correction step, the magnitude of the next stimulus and/or the parameters selected for stimulation in order to control a treatment device.

10. The method according to one of the preceding claims, wherein a separate data record is created for each medical aid, which can be edited after inputting an editing authorization.

11. A control unit for carrying out a method according to Claim 1, connected to an input portal for inputting the actual state of the body part to be corrected and to means for calculating a stimulus for the change of shape of a medical aid as well as to a memory, wherein the effect of the stimulus on the medical aid and the nominal position of the body part to be corrected are stored in the memory, **characterized in that** the memory of the control unit is designed to save the temporal course of correction steps.

12. The control unit according to Claim 11, wherein the control unit and the input portal are spatially separated, and wherein the data are transmitted from the input portal to the control unit.

13. The control unit according to Claim 11 or 12, wherein the input portal is opened by means of an access authorization.

## Revendications

1. Procédé de calcul d'un stimulus pour modifier la forme d'un instrument médical, de manière à modifier la forme de l'instrument à partir d'une position réelle jusqu'à un état de consigne définitif, comprenant les étapes suivantes :
détection de la position réelle de la partie de corps à corriger selon une première étape de correction dans une unité de commande,
comparaison de la position réelle de la partie de corps à corriger avec une position de consigne, laquelle est enregistrée dans l'unité de commande,
**caractérisé en ce que** le procédé présente en outre les étapes suivantes :
si nécessaire, détection de l'écart entre la position réelle et la position de consigne de la partie de corps à corriger par l'unité de commande,
calcul du stimulus censé agir sur l'instrument médical pour l'exécution d'une deuxième étape de correction, en tenant compte de l'écart entre l'état réel et l'état de consigne de la partie de corps à corriger par l'unité de commande, l'action du stimulus sur l'instrument médical étant enregistrée dans l'unité de commande.

2. Procédé selon la revendication 1, dans lequel le stimulus est sélectionné parmi un groupe comportant les paramètres de temps, de température, de valeur de pH, de courant, de rayonnement, de bruit, d'éclairage, ou de liquide ou dans lequel le stimulus est sélectionné à partir d'une combinaison de ces paramètres.

3. Procédé selon la revendication 1 ou 2, dans lequel la position réelle de la partie de corps à corriger est détectée par une empreinte, par une mesure numérique de la position de la partie de corps ou par des signaux d'un capteur détectant la position de la partie de corps.

4. Procédé selon l'une des revendications précédentes, dans lequel la position réelle de la partie de corps à corriger est obtenue par une entrée manuelle dans l'unité de commande ou par un portail d'entrée du fabricant de l'instrument médical ou par un portail d'entrée du médecin traitant.

5. Procédé selon l'une des revendications précédentes, dans lequel l'unité de commande est reliée à un dispositif de traitement, lequel est conçu pour laisser agir le stimulus calculé sur l'instrument médical.

6. Procédé selon l'une des revendications précédentes, dans lequel l'unité de commande enregistre l'évolution dans le temps de l'état réel de la partie de corps à corriger.

7. Procédé selon l'une des revendications précédentes, dans lequel l'unité de commande tient compte de l'évolution dans le temps des états réels de la partie de corps à corriger lors du calcul du stimulus pour la deuxième étape de correction.

8. Procédé selon l'une des revendications précédentes, dans lequel l'unité de commande calcule, annonce et enregistre la durée de la deuxième étape de correction, l'ordre de grandeur du stimulus suivant et/ou les paramètres sélectionnés pour la stimulation.

9. Procédé selon l'une des revendications précédentes, dans lequel la durée de la deuxième étape de correction annoncée par l'unité de commande, l'ordre de grandeur du stimulus suivant et/ou les paramètres sélectionnés pour la stimulation sont transmis manuellement à un dispositif de traitement ou dans lequel l'unité de commande utilise automatiquement la durée annoncée de la deuxième étape de correction, l'ordre de grandeur du stimulus suivant et/ou les paramètres sélectionnés pour la stimulation, pour la commande d'un dispositif de traitement.

10. Procédé selon l'une des revendications précédentes, dans lequel, pour chaque instrument médical, un jeu de données propre est établi, lequel est susceptible d'être traité après l'entrée d'une autorisation de traitement.

11. Unité de commande pour la mise en oeuvre d'un procédé selon la revendication 1, reliée à un portail d'entrée pour l'entrée de l'état réel de la partie de corps à corriger et à des moyens de calcul d'un stimulus permettant de modifier la forme d'un instrument médical et à une mémoire, dans laquelle l'action du stimulus sur l'instrument médical et la position de consigne de la partie de corps à corriger sont enregistrées dans la mémoire, **caractérisée en ce que** la mémoire de l'unité de commande est conçue pour enregistrer l'évolution dans le temps d'étapes de correction.

12. Unité de commande selon la revendication 11, dans laquelle l'unité de commande et le portail d'entrée sont séparés spatialement, et dans laquelle les données sont transmises du portail d'entrée à l'unité de commande.

13. Unité de commande selon la revendication 11 ou 12, dans laquelle le portail d'entrée est ouvert au moyen d'une autorisation d'accès.
